(19) Europäisches Patentamt
European Patent Office
Office européen des brevets

(11) Veröffentlichungsnummer: **0 155 486**
**A1**

(12) # EUROPÄISCHE PATENTANMELDUNG

(21) Anmeldenummer: **85101280.7**

(22) Anmeldetag: **07.02.85**

(51) Int. Cl.[4]: **C 07 D 249/12**
C 07 D 409/04, C 07 D 401/12
A 61 K 31/41, A 61 K 31/44
//(C07D409/04, 333:00, 249:00),
(C07D401/12, 249:00, 213:00)

(30) Priorität: **07.02.84 HU 48884**

(43) Veröffentlichungstag der Anmeldung:
**25.09.85 Patentblatt 85/39**

(84) Benannte Vertragsstaaten:
**AT BE CH DE FR GB LI NL SE**

(72) Erfinder: **Nagy, Gábor**
**Fadrusz u. 6**
**H-1114 Budapest(HU)**

(72) Erfinder: **Janáky, Judit, Dr.-Pharm.**
**Frankel Leó u. 102-104**
**H-1023 Budapest(HU)**

(72) Erfinder: **Andrási, Ferenc, Dr.-Phys.**
**Kutvölgyi u. 69**
**H-1125 Budapest(HU)**

(71) Anmelder: **ALKALOIDA VEGYéSZETI GYáR**
**Postfach 1**
**H-4440 Tiszavasvári(HU)**

(72) Erfinder: **Somorai, Tamás, Dr.-Chem.**
**Alvinci u. 27**
**H-1022 Budapest(HU)**

(72) Erfinder: **Szilágyi, Géza, Dr.-Chem.**
**Szakasits A. u. 66/a**
**H-1115 Budapest(HU)**

(72) Erfinder: **Reiter, József, Dr.-Chem.**
**Mihályfi E. u. 32/b**
**H-1022 Budapest(HU)**

(72) Erfinder: **Bozó, Eva**
**VIII. u. 22**
**H-1172 Budapest(HU)**

(74) Vertreter: **Beszédes, Stephan G. Dr.**
**Münchener Strasse 80a Postfach 1168**
**D-8060 Dachau(DE)**

(54) **1,3,5-Trisubstituierte 1,2,4-Triazolderivate, Verfahren zu ihrer Herstellung und diese Verbindungen enthaltende Arzneimittel.**

(57) Gegenstand der Erfindung sind 1,3,5-trisubstituierte 1,2,4-Triazolderivate der allgemeinen Formel

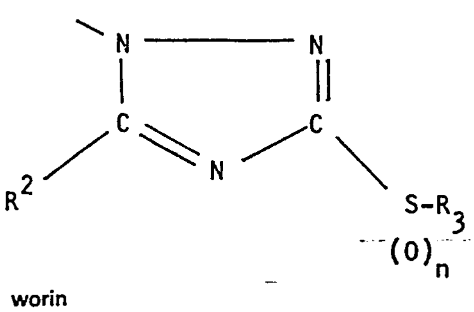

I

worin

$R_1$ für einen, gegebenenfalls durch 1 oder mehr Fluor-, Chlor- und/oder Bromatom(e), Alkylrest(e), Alkoxyrest(e), Nitrogruppe(n), Trifluormethylrest(e), Alkylthiorest(e), Alkylsulfinylrest(e) und/oder Alkylsulfonylrest(e) substituierten, Phenylrest oder einen Naphthylrest steht,

$R_2$ einen, gegebenenfalls durch 1 oder mehr Fluor-, Chlor- und/oder Bromatom(e), Alkylrest(e), Alkoxyrest(e), Nitrogruppe(n), Trifluormethylrest(e), Alkylthiorest(e), Alkylsulfinylrest(e) und/oder Alkylsulfonylrest(e) substituierten, Phenylrest oder einen Thienylrest bedeutet,

$R_3$ einen, gegebenenfalls durch 1 oder mehr Fluor- und/oder Chloratom(e), Hydroxygruppe(n), Alkoxycarbonylrest(e), Cyangruppe(n) und/oder Pyridylrest(e) substituierten, Alkylrest, einen Alkoxycarbonylrest, einen Oxoalkylrest oder einen im Phenylring durch 1 oder mehr Fluor- und/oder Chloratom(e) substituierten Phenylalkylrest darstellen und

n 0, 1 oder 2 ist.

Gegenstand der Erfindung sind auch ein Verfahren zur Herstellunt dieser Verbindungen und diese enthaltende Arzneimittel, insbesondere mit entzündungshemmende und antirheumatischer Wirkung.

EP 0 155 486 A1

Croydon Printing Company Ltd.

Die Erfindung betrifft neue 1,3,5-trisubstituierte 1,2,4-Triazolderivate, ein Verfahren zu ihrer Herstellung und diese Verbindungen enthaltende Arzneimittel, insbesondere mit entzündungshemmender und antirheumatischer Wirkung.

Es ist bereits 1,5-Bis-[phenyl]-3-[methylthio]-1,2,4-triazol aus dem Schrifttum (Augustin und Mitarbeiter: J. f. prakt. Chemie 322 [1980], Seiten 55 bis 68) bekannt, von einer pharmakologischen Wirkung desselben ist jedoch keine Rede.

Der Erfindung liegt die Aufgabe zugrunde, überlegene pharmakologische Wirkungen aufweisende neue 1,2,4-Triazolderivate, ein Verfahren zur Herstellung derselben und diese Verbindungen enthaltende Arzneimittel zu schaffen.

Das Obige wurde überraschenderweise durch die Erfindung erreicht.

Gegenstand der Erfindung sind 1,3,5-trisubstituierte 1,2,4-Triazolderivate der allgemeinen Formel

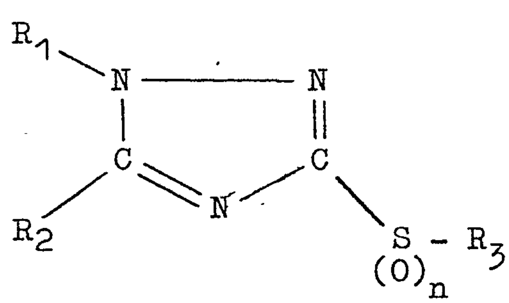

I ,

worin

$R_1$ für einen, gegebenenfalls durch 1 oder mehr

Fluor-, Chlor- und/oder Bromatom(e), Alkylrest(e) mit 1 bis 4 Kohlenstoffatom(en), Alkoxyrest(e) mit 1 bis 4 Kohlenstoffatom(en), Nitrogruppe(n), Trifluormethylrest(e), Alkylthiorest(e) mit 1 bis 4 Kohlenstoffatom(en), Alkylsulfinylrest(e) mit 1 bis 4 Kohlenstoffatom(en) und/oder Alkylsulfonylrest(e) mit 1 bis 4 Kohlenstoffatom(en) substituierten, Phenylrest oder einen Naphthylrest steht,

$R_2$ einen, gegebenenfalls durch 1 oder mehr Fluor-, Chlor- und/oder Bromatom(e), Alkylrest(e) mit 1 bis 4 Kohlenstoffatom(en), Alkoxyrest(e) mit 1 bis 4 Kohlenstoffatom(en), Nitrogruppe(n), Trifluormethylrest(e), Alkylthiorest(e) mit 1 bis 4 Kohlenstoffatom(en), Alkylsulfinylrest(e) mit 1 bis 4 Kohlenstoffatom(en) und/oder Alkylsulfonylrest(e) mit 1 bis 4 Kohlenstoffatom(en) substituierten, Phenylrest oder einen Thienylrest bedeutet,

$R_3$ einen, gegebenenfalls durch 1 oder mehr Fluor- und/oder Chloratom(e), Hydroxygruppe(n), Alkoxycarbonylrest(e) mit 2 bis 4 Kohlenstoffatomen, Cyangruppe(n) und/oder Pyridylrest(e) substituierten, Alkylrest mit 1 bis 4 Kohlenstoffatom(en), einen Alkoxycarbonylrest mit 2 bis 4 Kohlenstoffatomen, einen Oxoalkylrest mit 2 bis 5 Kohlenstoffatomen oder einen im Phenylring durch 1 oder mehr Fluor- und/oder Chloratom(e) substituierten Phenylalkylrest mit 1 bis 4 Kohlenstoffatom(en) im Alkylteil darstellen und

n 0, 1 oder 2 ist,

mit der weiteren Maßgabe, daß,

im Falle daß

n O ist und

$R_3$ einen Methylrest bedeutet,

$R_1$ und $R_2$ nicht beide einen nicht substituierten Phenylrest darstellen.

Im Falle, daß $R_1$ und/oder $R_2$ für [einen] substituierte[n] Phenylrest(e) steht beziehungsweise stehen, ist die Zahl des beziehungsweise der Substituenten vorzugsweise 1 oder 2, insbesondere 1,

Vorzugsweise ist beziehungsweise sind der beziehungsweise die Alkylrest(e), Alkoxyrest(e), Alkylthiorest(e), Alkylsulfinylrest(e) und/oder Alkylsulfonylrest(e), durch welche[n] der beziehungsweise die Phenylrest(e), für den beziehungsweise die $R_1$ und/oder $R_2$ stehen kann beziehungsweise können, substituiert sein kann beziehungsweise können, [ein] solche[r] mit 1 bis 3, insbesondere 1 oder 3, ganz besonders 1, Kohlenstoffatom(en). Im Falle, daß der beziehungsweise die Phenylrest(e), für den beziehungsweise die $R_1$ und/oder $R_2$ stehen kann beziehungsweise können, durch [einen] Alkylthiorest(e) mit 3 Kohlenstoffatomen substituiert ist beziehungsweise sind, ist beziehungsweise sind der beziehungsweise die letztere(n) bevorzugt [ein] Isopropylthiorest(e).

Es ist auch bevorzugt, daß sich der beziehungsweise die Substituiert(en) im Falle von substituierten Phenylresten, für welche $R_1$ stehen kann, in der beziehungsweise den 4-, 3- oder 3,4-Stellung(en) und im Falle von substituierten

Phenylresten, welche $R_2$ bedeuten kann, in der beziehungsweise den 4-, 2- oder 3,4-Stellung(en) befindet beziehungsweise befinden. In den substituierten Phenylresten, für welche $R_1$ und/oder $R_2$ stehen kann beziehungsweise können, ist im Falle des Vorliegens von 1 Substituenten dieser besonders bevorzugt in der 4-Stellung.

Besonders bevorzugte Reste, für welche $R_1$ und/oder $R_2$ stehen kann beziehungsweise können, sind Methylthiophenyl-, Isopropylthiophenyl-, Methylphenyl-, Methoxyphenyl-, Trifluormethylphenyl-, Methylsulfonylphenyl- und Dichlorphenylreste sowie Naphthylreste, vor allem der Naphth-2-ylrest, und Thienylreste, vor allem der Thien-2-ylrest.

Es ist auch bevorzugt, daß der Alkylrest, für welchen $R_3$ stehen kann, ein solcher mit 1 bis 3, insbesondere 1 oder 2, Kohlenstoffatom(en) ist.

Ferner ist es bevorzugt, daß der Alkoxycarbonylrest, für den $R_3$ stehen kann, beziehungsweise der beziehungsweise die Alkoxycarbonylrest(e), durch welche[n] der Alkylrest, für den $R_3$ stehen kann, substituiert sein kann, [ein] solche[r] mit 2 oder 3, insbesondere 2, Kohlenstoffatomen ist beziehungsweise sind.

Weiterhin ist es bevorzugt, daß der Oxoalkylrest, für den $R_3$ stehen kann, ein solcher mit 2 bis 4, insbesondere 2 oder 3, ganz besonders 3, Kohlenstoffatomen ist.

Außerdem ist es bevorzugt, daß der Phenylalkylrest, für welchen $R_3$ stehen kann, ein solcher mit 1 bis 3, insbesondere 1 oder 2, ganz besonders 1, Kohlenstoffatom(en) im Alkylteil ist.

Im Falle, daß der Alkylrest beziehungsweise Oxoalkylrest, für den $R_3$ stehen kann, ein solcher mit 3 Kohlenstoffatomen ist, ist er bevorzugt ein Propylrest beziehungsweise Oxopropylrest, vor allem 2-(Oxo)-propylrest.

Vorzugsweise ist beziehungsweise sind der beziehungsweise die Pyridylrest(e), durch welche[n] der Alkylrest, für den $R_3$ stehen kann, substituiert sein kann, [ein] Pyrid-2--yl- und/oder Pyrid-3-ylrest(e).

Im Falle, daß $R_3$ für einen durch 1 oder mehr Fluor- und/oder Chloratom(e) substituierten Alkylrest steht, ist die Zahl der Substituenten vorzugsweise 3 oder 4. Bevorzugt befinden sie sich im Falle, daß es sich um einen substituierten Äthylrest handelt, in den 1,1,2-, 2,2,2- beziehungsweise 1,1,2,2-Stellungen und im Falle, daß es sich um einen substituierten Propylrest handelt, in den 2,2,3,3-Stellungen.

Im Falle, daß $R_3$ für einen im Phenylring durch 1 oder mehr Fluor- und/oder Chloratom(e) substituierten Phenylalkylrest steht, ist die Zahl des beziehungsweise der Substituenten vorzugsweise 1 oder 2, insbesondere 2. Er beziehungsweise sie befindet beziehungsweise befinden sich bevorzugt in der beziehungsweise den 3- und/oder 4-Stellung(en).

Besonders bevorzugte Reste, für welche $\underset{(O)_n}{-S-}R_3$ stehen kann, sind Methylthio-, Äthylthio-, Propylthio-, Pyridylmethylthio-, vor allem Pyrid-2-ylmethylthio- beziehungsweise Pyrid-3-ylmethylthio-, Pyridyläthylthio-, vor allem Pyrid-2-yläthylthio- beziehungsweise Pyrid-3-yläthylthio-, Cyanmethylthio-, 1-(Äthoxycarbonyl)-äth-1-ylthio-, Dichlorbenzylthio-, vor allem 3,4-Di-(chlor)-benzylthio-, Fluorbenzylthio-, vor allem 4-(Fluor)-benzylthio-, Hydroxy-

äthylthio-, vor allem 2-(Hydroxy)-äthylthio-, Äthoxycarbonylmethylthio-, Oxopropylthio-, vor allem 2-(Oxo)-propylthio-, Methoxycarbonylthio-, Äthoxycarbonylthio-, Trifluoräthylthio-, vor allem 2,2,2-Tri-(fluor)-äthylthio-, Chlortrifluoräthylthio-, vor allem 2-(Chlor)-1,1,2-tri-(fluor)-äthylthio-, Tetrafluoräthylthio-, vor allem 1,1,2,2-Tetra-(fluor)-äthylthio-, Tetrafluorpropylthio-, vor allem 2,2,3,3-Tetra-(fluor)-propylthio-, Methylsulfinyl-, Methylsulfonyl-, Trifluoräthylsulfonyl-, vor allem 2,2,2-Tri-(fluor)-äthylsulfonyl-, Tetrafluoräthylsulfonyl-, vor allem 1,1,2,2-Tetra-(fluor)-äthylsulfonyl-, Trifluorchloräthylsulfonyl-, vor allem 1,1,2-Tri-(fluor)-2-(chlor)-äthylsulfonyl-, und Pyridylmethylsulfonyl-, vor allem Pyrid-2-ylmethylsulfonyl- beziehungsweise Pyrid-3-ylmethylsulfonylreste.

Besonders bevorzugte erfindungsgemäße 1,2,4-Triazolderivate sind 1,5-Bis-[4'-(chlor)-phenyl]-3-[methylthio]-1,2,4-triazol, 1-[4'-(Chlor)-phenyl]-5-[4''-(fluor)-phenyl]-3-[methylthio]-1,2,4-triazol, 1-[4'-(Fluor)-phenyl]-5-[4''-(chlor)-phenyl]-3-[methylthio]-1,2,4-triazol, 1-[4'-(chlor)-phenyl]-5-[4''-(fluor)-phenyl]-3-[methylsulfonyl]-1,2,4-triazol und 1,5-Bis-[4'-(fluor)-phenyl]-3-[methylsulfonyl]-1,2,4-triazol.

Gegenstand der Erfindung ist auch ein Verfahren zur Herstellung der erfindungsgemäßen Verbindungen, welches dadurch gekennzeichnet ist, daß

a) 1,5-disubstituierte 1,2,4-Triazolin-3[2H]-thione {2,3-Dihydro-1,2,4-triazol-3-thione} der allgemeinen Formel

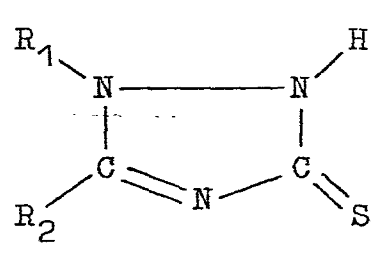

II ,

worin $R_1$ und $R_2$ die oben angegebenen Bedeutungen haben, oder 1-substituierte 1-(Acyl)-thiosemicarbazide der allgemeinen Formel

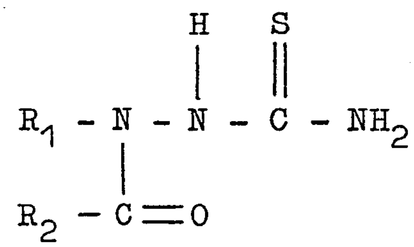

V ,

worin $R_1$ und $R_2$ die oben angegebenen Bedeutungen haben, in Lösung in inerten Lösungsmitteln in Gegenwart von organischen und/oder anorganischen Basen im alkalischen pH-Bereich mit Verbindungen der allgemeinen Formel

$$R_3 - X \qquad \text{III} ,$$

worin $R_3$ die oben angegebenen Bedeutungen hat und X ein Halogenatom oder einen Toluolsulfonyloxyrest [Tosyloxyrest] bedeutet, umgesetzt werden und gegebenenfalls die erhaltenen 1,2,4-Triazolderivate der allgemeinen Formel I, bei welchen n 0 ist, zu 1,2,4-Triazolderivaten der allgemeinen Formel I, bei welchen n 1 oder 2 ist, oxydiert werden oder

b) zur Herstellung derjenigen erfindungsgemäßen 1,2,4-Triazolderivate der allgemeinen Formel I, bei welchen $R_3$ für einen durch 1 oder mehr Fluor- und/oder Chloratom(e) substituierten Äthylrest steht und n 0 ist, Tetrahalogenäthylene der allgemeinen Formel

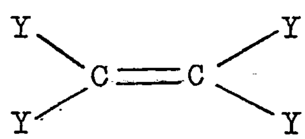

IV ,

worin Y unabhängig voneinander Fluor- und/oder Chloratome bedeuten, an 1,5-disubstituierte 1,2,4-Triazolin-3-[2H]-thione der allgemeinen Formel II oder 1-substituierte 1-(Acyl)-thiosemicarbazide der allgemeinen Formel V in inerten Lösungsmitteln in Gegenwart von organischen und/oder anorganischen Basen im alkalischen pH-Bereich angelagert werden oder

c) zur Herstellung derjenigen erfindungsgemäßen 1,2,4-Triazolderivate der allgemeinen Formel I, bei welchen $R_3$ für einen Alkylrest steht und n 0 ist, 1,5-disubstituierte 1,2,4-Triazolin-3[2H]-thione der allgemeinen Formel II oder 1-substituierte 1-(Acyl)-thiosemicarbazide der allgemeinen Formel V in inerten Lösungsmitteln in Gegenwart von organischen und/oder anorganischen Basen im alkalischen pH-Bereich mit Diazomethan oder Dialkylsulfaten mit 1 bis 4 Kohlenstoffatom(en) alkyliert werden.

Gemäß einer vorteilhaften Ausführungsform der Variante a) des erfindungsgemäßen Verfahrens wird 1 Mol eines 1,5-disubstituierten 1,2,4-Triazolin-3[2H]-thiones der allgemeinen Formel II in Lösung in einer 1,2 bis 2,2 Mol Natriumhydroxyd enthaltenden wäßrigen Natriumhydroxydlösung in Gegenwart eines niederen Alkoholes, vorzugsweise von Methanol oder Äthanol, bei Temperaturen von -50 bis 100°C, vorzugsweise von -10°C bis 80°C, mit 1 bis 3 Mol einer Verbindung der allgemeinen Formel III umgesetzt und nach beendeter Umsetzung das erhaltene 1,2,4-Triazolderivatprodukt der allgemeinen Formel I nach einer bekannten Verfahrensweise isoiert.

Nach einer anderen vorteilhaften Ausführungsform der Variante a) des erfindungsgemäßen Verfahrens wird eine Lösung von 1 Mol eines 1-substituierten 1-(Acyl)-thiosemicarbazides der allgemeinen Formel V in einer

1,5 bis 2,5 Mol Natriumhydroxyd enthaltenden wäßrigen Natriumhydroxydlösung bei einem pH-Wert über 7,5 in Gegenwart eines niederen Alkoholes, vorzugsweise von Methanol oder Äthanol, bei Temperaturen von -10 bis 80°C mit 1,0 bis 1,1 Mol einer Verbindung der allgemeinen Formel III umgesetzt und nach beendeter Umsetzung das 1,2,4-Triazolderivatprodukt der allgemeinen Formel I nach an sich bekannten Verfahrensweisen isoliert.

Gemäß einer vorteilhaften Ausführungsform der Variante b) des erfindungsgemäßen Verfahrens wird in eine Lösung eines 1,5-disubstituierten 1,2,4-Triazolin-3[2H]-thiones der allgemeinen Formel II in einem aprotischen organischen Lösungsmittel, vorzugsweise Dimethylformamid, in Gegenwart von Triäthylamin oder Benzyltrimethylammoniumhydroxyd (Triton® B) bei Temperaturen von -20 bis 60°C, vorzugsweise 0 bis 20°C, Tetrafluoräthylen oder Trifluorchloräthylen geleitet, die dann 1 Stunde lang bei 40 bis 50°C gerührt beziehungsweise geschüttelt wird. Nach beendeter Umsetzung wird das 1,2,4-Triazolderivatprodukt der allgemeinen Formel I, bei welchem $R_3$ einen Tetrafluoräthyl- oder Trifluorchloräthylrest bedeutet, nach einer bekannten Verfahrensweise isoliert.

Nach einer vorteilhaften Ausführungsform der Variante c) des erfindungsgemäßen Verfahrens werden einer Lösung von 1 Mol eines 1,5-disubstituierten 1,2,4-Triazolin-3[2H]--thiones der allgemeinen Formel II oder eines 1-substituierten 1-(Acyl)-thiosemicarbazides der allgemeinen Formel V in einer 2,0 bis 3,5 Mol Natriumhydroxyd enthaltenden wäßrigen Natriumhydroxydlösung 1 bis 1,5 Mol Dimethyl- oder Diäthylsulfat zugetropft. Nach beendeter Umsetzung wird das 1,2,4-Triazolderivatprodukt der allgemeinen Formel I, bei welchem $R_3$ einen Methyl- oder Äthylrest bedeutet, nach einer

an sich bekannten Verfahrensweise isoliert.

Nach einer anderen vorteilhaften Ausführungsform der Variante c) des erfindungsgemäßen Verfahrens zur Herstellung der 1,2,4-Triazolderivate der allgemeinen Formel I, bei welchen $R_3$ einen Methylrest bedeutet, wird eine Suspension eines 1,5-disubstituierten 1,2,4-Triazolin-3[2H]-thiones der allgemeinen Formel II in einem inerten Lösungsmittel, vorzugsweise Äther, bei Temperaturen von 0 bis 30°C mit einer ätherischen Lösung von Diazomethan versetzt und das 1,2,4-Triazolderivatprodukt der allgemeinen Formel I nach beendeter Umsetzung nach einer an sich bekannten Verfahrensweise isoliert.

Die gegebenenfalls erfolgende Oxydation der nach dem erfindungsgemäßen Verfahren hergestellten 1,3,5-trisubstituierten 1,2,4-Triazolderivate der allgemeinen Foreml I, bei welchen n 0 ist, wird vorzugsweise mit m-Chlorperbenzoesäure oder Peressigsäure durchgeführt. Bei Verwendung von 1,0 bis 1,1 Mol m-Chlorperbenzoesäure oder Peressigsäure entsteht das entsprechende Monoxyd, nämlich Sulfoxyd (n = 1), und bei Verwendung von 2,0 bis 2,2 Mol m-Chlorperbenzoesäure oder Peressigsäure wird das entsprechende Dioxyd, nämlich Sulfon (n = 2) erhalten. Die Persäureoxydation wird vorzugsweise bei 0°C in wasserfreiem Chloroform durchgeführt. Zur Herstellung von Sulfoxyden (n = 1) kann als andere bevorzugte Möglichkeit als Oxydationsmittel auch Brom in wäßrigem Alkali bei Raumtemperatur eingesetzt werden. Es ist zweckmäßig, die Bromoxydation in einem Zweiphasensystem, wie in einem Gemisch aus einem Halogen gebunden enthaltenen Lösungsmittels vorzugsweise Dichlormethan, und einer wäßrigen Kaliumbicarbonatlösung, durchzuführen. Die Oxydation des Sulfoxydes selbst kann durch Zugabe von 5 bis 10 Gew.-% Dimethylsulfoxyd zur Reaktionslösung verhindert werden.

Die Verfahren zur Herstellung der im erfindungsgemäßen

Verfahren als Ausgangssubstanzen verwendbaren 1,5-disubstituierten 1,2,4-Triazolin-3[2H]-thione der allgemeinen Formel II sind aus dem Schrifttum (J. Chem. Soc., Perkin Trans. I., 1979, Seite 724) bekannt.

Die Verbindungen der allgemeinen Formel III und die Tetrahalogenäthylene der allgemeinen Formel IV sind bekannte Handelsprodukte.

Die 1-substituierten 1-(Acyl)-thiosemicarbazide der allgemeinen Formel V sind teilweise bekannte und teilweise neue Verbindungen und können vorzugsweise wie folgt hergestellt worden sein. Es wird ein 1-substituiertes Thiosemicarbazid der allgemeinen Formel

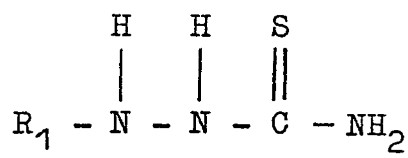

VI ,

worin $R_1$ die obigen Bedeutungen hat (zum größten Teil aus dem Schrifttum, zum Beispiel Chem. Ber., 28, [1895], Seite 2 081, Gazette 28, II. Seite 560, J. Indian Chem. Soc., 5, Seite 653 und C. Z. 1929, I. Seite 1 110, bekannt) mit der äquimolaren Menge eines Säurehalogenides der allgemeinen Formel

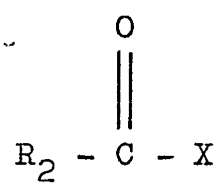

VII ,

worin $R_2$ und X die obigen Bedeutungen, ausgenommen beim letzteren die Toluolsulfonyloxygruppe, ohne oder in Lösungsmittel(n), vorzugsweise in Benzol, bei Temperaturen von 0 bis 150°C, vorzugsweise 30 bis 90°C, umgesetzt. Die Umsetzung kann auch in Gegenwart eines säurebindenden Mittels durchgeführt werden, was besonders bei Verbindungen, die eine säureempfindliche Gruppe, zum Beispiel Methoxygruppe, haben, günstig ist.

Ferner sind erfindungsgemäß Arzneimittel, welche 1 oder mehr der erfindungsgemäßen Verbindungen als Wirkstoff(e), zweckmäßigerweise zusammen mit 1 oder mehr üblichen inerten nicht toxischen festen oder flüssigen Trägerstoff(en), Hilfsmittel(n) und/oder Zusatzstoff(en), enthalten, vorgesehen.

Die erfindungsgemäßen 1,2,4-Triazolderivate haben nämlich wertvolle therapeutsche Wirkungen, insbesondere antirheumatische und entzündungshemmende Wirkungen, wobei sie eine geringe Toxizität aufweisen.

Die Untersuchung der therapeutischen Wirkung der erfindungsgemäßen 1,2,4-Triazolderivate wurde mittels des modifizierten Newbould-Prüfversuches (Brit. J. Pharmacol., 21 [1963], Seite 127) durchgeführt. Dazu wurde in die rechen Hinterfüße von männlichen Ratten der Art Long Evans mit Gewichten von 180 bis 250 g intraplantar 0,25 g abgetötetes Mycobacterium tuberculosis in einem Volumen von 0,1 ml injiziert. Nachfolgend wurden die Ratten pro betreffenden Tag mit einer Dosis der zu untersuchenden Verbindung insgesamt 16-mal während 3 Wochen behandelt. Die Änderung des Volumens der Fußschwellung wurde 21 Tage lang täglich mit einem Quecksilber-Plethysmometer vom Lence-Typ bestimmt. Vom 10-ten Tag an wurde das Volumen des linken Hinterfußes bestimmt. Die prozentuale Hemmung wurde im Verhältnis zu diesem Volumen aus der eintretenden Änderung in der Schwellung des rechten Hinterfußes berechnet. Es wurden auch die entsprechenden Werte der anerkannt gut wirksamen Vergleichssubstanzen 4-(Butyl)-1,2-di-(phenyl)-pyrazolidin-3,5-dion {Phenylbutazon} ⟨Vergleichssubstanz A⟩ und (+)-2-[6'-(Methoxy)-naphth-2'-yl]-propionsäure {Naproxen} ⟨Vergleichssubstanz B⟩ gleicher Wirkungsrichtung ermittelt. Die Hemmungswerte, Indikatoren der antirheumatischen Wirkung, sind in der folgenden Tabelle 1 zusammengestellt.

Tabelle 1

Antirheumatische Wirkung

| Verbindung | | Prozentuale Hemmung bei einer peroralen Dosis von | | | | Peroraler $ED_{50}$-Wert in mg/kg an Ratten | Peroraler $LD_{50}$-Wert in mg/kg an Ratten | Therapeutischer Index $\frac{LD_{50}\text{-Wert}}{ED_{50}\text{-Wert}}$ |
|---|---|---|---|---|---|---|---|---|
| Beispiel Nr. | Bezeichnung | 1,56 mg/kg | 3,12 mg/kg | 6,25 mg/kg | 12,5 mg/kg | | | |
| 4 | 1,5-Bis-[4'-(chlor)-phenyl]-3-[pyrid-2''-ylmethylthio]-1,2,4-triazol | | -38 | -40 | | | | |
| 14 | 1-[4'-(Chlor)-phenyl]-5-[2''-(methylthio)-phenyl]-3-[methylthio]-1,2,4-triazol | | -5 | | -51 | | | |
| 27 | 1,5-Bis-[4'-(chlor)-phenyl]-3-[methylthio]-1,2,4-triazol | -17 | -39 | -55 | -67 | 7,4 | 480 | 64,9 |
| 30 | 1-[4'-(Chlor)-phenyl]-5-[thien-2''-yl]-3-[methylthio]-1,2,4-triazol | | | | -44 | | | |

Fortsetzung der Tabelle 1

| Verbindung Beispiel Nr. | Bezeichnung | Prozentuale Hemmung bei einer peroralen Dosis von 1,56 mg/kg | 3,12 mg/kg | 6,25 mg/kg | 12,5 mg/kg | Peroraler $ED_{50}$-Wert in mg/kg an Ratten | Peroraler $LD_{50}$-Wert in mg/kg an Ratten | Therapeutischer Index $\frac{LD_{50}\text{-Wert}}{ED_{50}\text{-Wert}}$ |
|---|---|---|---|---|---|---|---|---|
| 41 | 1-[4'-(Fluor)-phenyl]-5-[4''-(fluor)-phenyl]-3-[methylthio]-1,2,4-triazol | | -42 | -57 | | | | |
| 42 | 1-[3'-(Trifluormethyl)-phenyl]-5-[4"-(chlor)-phenyl]-3-[methylthio]-1,2,4-triazol | | | -48 | | | | |
| 44 | 1-[4'-(Chlor)-phenyl]-5-[4"-(fluor)-phenyl]-3-[methylthio]-1,2,4-triazol | -36 | -64 | -53 | | 2,1 | 170 | 81,0 |
| 45 | 1-[4'-(Fluor)-phenyl]-5-[4"-(trifluormethyl)-phenyl]-3-[methylthio]-1,2,4-triazol | | | -25 | -48 | | | |



0155486

Fortsetzung der Tabelle 1

| Verbindung Beispiel Nr. | Bezeichnung | Prozentuale Hemmung bei einer peroralen Dosis von 1,56 mg/kg | 3,12 mg/kg | 6,25 mg/kg | 12,5 mg/kg | Peroraler $ED_{50}$-Wert in mg/kg an Ratten | Peroraler $LD_{50}$-Wert in mg/kg an Ratten | Therapeutischer Index $\frac{LD_{50}\text{-Wert}}{ED_{50}\text{-Wert}}$ |
|---|---|---|---|---|---|---|---|---|
| 47 | 1-[3'-(Trifluormethyl)-phenyl]-5-[4"-(fluor)-phenyl]-3-[methylthio]-1,2,4-triazol | | | -22 | -53 | | | |
| 53 | 1-[3'-(Chlor)-phenyl]-5-[4"-(fluor)-phenyl]-3-[methylthio]-1,2,4-triazol | | | | -51 | | | |
| 54 | 1-[4'-(Brom)-phenyl]-5-[4"-(chlor)-phenyl]-3-[methylthio]-1,2,4-triazol | | | -28 | -56 | | | |
| 55 | 1-[4'-(Fluor)-phenyl]-5-[4"-(chlor)-phenyl]-3-[methylthio]-1,2,4-triazol | -12 | -26 | -68 | | 6,8 | 380 | 55,9 |

0155486

Fortsetzung der Tabelle 1

| Verbindung Beispiel Nr. | Bezeichnung | Prozentuale Hemmung bei einer peroralen Dosis von 1,56 mg/kg | 3,12 mg/kg | 6,25 mg/kg | 12,5 mg/kg | Peroraler $ED_{50}$-Wert in mg/kg an Ratten | Peroraler $LD_{50}$-Wert in mg/kg an Ratten | Therapeutischer Index $\frac{LD_{50}\text{-Wert}}{ED_{50}\text{-Wert}}$ |
|---|---|---|---|---|---|---|---|---|
| 59 | 1,5-Bis-[4'-(fluor)-phenyl]-3-[1",1",2",2"-tetra-(fluor)-äthylthio]-1,2,4-triazol | | -44 | | | | | |
| 65 | 1-[4'-(Chlor)-phenyl]-5-[4"-(fluor)-phenyl]-3-[methylsulfonyl]-1,2,4-triazol | | -43 | -68 | | | | |
| 66 | 1-[4'-(Fluor)-phenyl]-5-[4"-(chlor)-phenyl]-3-[methylsulfonyl]-1,2,4-triazol | | | -54 | | | | |
| 67 | 1,5-Bis-[4'-(fluor)-phenyl]-3-[methylsulfonyl]-1,2,4-triazol | | -58 | | | | | |

Fortsetzung der Tabelle 1

| Verbindung Beispiel Nr. | Bezeichnung | Prozentuale Hemmung bei einer peroralen Dosis von 1,56 mg/kg | 3,12 mg/kg | 6,25 mg/kg | 12,5 mg/kg | Peroraler $ED_{50}$-Wert in mg/kg an Ratten | Peroraler $LD_{50}$-Wert in mg/kg an Ratten | Therapeutischer Index $\frac{LD_{50}\text{-Wert}}{ED_{50}\text{-Wert}}$ |
|---|---|---|---|---|---|---|---|---|
| Vergleichssubstanz A | 4-(Butyl)-1,2-di-(phenyl)-pyrazolidin-3,5-dion {Phenylbutazon} | | | | -7 | | | |
| Vergleichssubstanz B | (+)-2-[6'-(Methoxy)-naphth-2'-yl]-propionsäure {Naproxen} | | | | -34 *) | 12,5 | 543 | 43,4 |

*) Dosis: 10 mg/kg

Aus der obigen Tabelle 1 geht hervor, daß die erfindungsgemäßen 1,2,4-Triazolderivate gegenüber den Vergleichsubstanzen eine überlegene antirheumatische Wirkung bei Berücksichtigung auch ihrer geringen Toxizität haben.

Auch die geschwürbildende beziehungsweise ulcerogene Wirkung, eine der kritischsten Nebenwirkungen entzündungshemmender Mittel, betreffend erwiesen sich die erfindungsgemäßen 1,2,4-Triazolderivate als im Vergleich zu Handelspräparaten günstiger. So führten Einzeldosen von 15 mg/kg 1-(p-Chlorbenzoyl)-5-(methoxy)-2-(methyl)-indol-3-ylessigsäure {Indometacin} bei 100% der Ratten Geschwüre (Ulcus) herbei, während Einzeldosen von 1 000 mg/kg 1,5-Bis-[4'-(chlor)-phenyl]-3-[methylthio]-1,2,4-triazol (Verbindung des Beispieles 27) nur bei 10% der Tiere Geschwüre verursachten. Selbst bei peroraler Dauerbehandlung der Ratten mit der letzteren wurde bei Dosen von 16 x 20 mg/kg nur in Einzelfällen Bluterguß (Suffusion) nachgewiesen.

Die obigen Versuchsergebnisse zeigen, daß die erfindungsgemäßen 1,2,4-Triazolderivate wirksamer sind als die in der gegenwärtigen Therapie mit Erfolg eingesetzten Vergleichsverbindungen 4-(Butyl)-1,2-di-(phenyl)-pyrazolidin-3,5-dion {Phenylbutazon} ⟨Vergleichssubstanz A⟩ und (+)-2-[6'-(Methoxy)-naphth-2'-yl]-propionsäure {Naproxen} ⟨Vergleichssubstanz B⟩ und außerdem haben sie den Vorteil gegenüber den Vergleichsverbindungen, daß sie im verwendeten Dosisbereich weder bei akuter noch bei chronischer Verabreichung eine geschwürbildende beziehungsweise ulcerogene Nebenwirkung ausüben. Das ist besonders vorteilhaft, da die Antirheumatika meistens für eine Dauerbehandlung eingesetzt werden.

Nach eigenen Beobachtungen sind die erfindungsgemäßen

1,2,4-Triazolderivate der allgemeinen Formel I Antirheumatica, in deren Wirkungsmechanismus auch ihre Immunmodulatoreigenschaft eine Rolle spielt.

Die erfindungsgemäßen Arzneimittel können in Form von Arzneimittelpräparaten vorliegen.

Die erfindungsgemäßen Arzneimittelpräparate können nach üblichen an sich bekannten Verfahrensweisen hergestellt werden.

Falls es erwünscht ist, können die erfindungsgemäßen Arzneimittel auch noch weitere Wirkstoffe, besonders Antirheumatica, enthalten.

Die erfindungsgemäßen 1,2,4-Triazolderivate können in einer täglichen Dosis von 2 bis 6 mg/kg verabreicht werden. Die Dosierung wird vorzugsweise dem Gewicht, dem Alter und dem allgemeinen Gesundheitszustand des Patienten angepaßt.

Die Erfindung wird an Hand der folgenden Beispiele näher erläutert.

Beispiel 1

1,5-Bis-[4'-(chlor)-phenyl]-3-
-[propylthio]-1,2,4-triazol

Es wurde ein Gemisch von 3,0 g (9,3 mMol) 1,5-Bis-(4'-chlor)-phenyl]-1,2,4-triazolin-3[2H]-thion, 10 ml einer 0,48 g (12 mMol) Natriumhydroxyd enthaltenden Natriumhydroxydlösung, 20 ml Äthanol und 1,59 g (9,3 mMol) Propyljodid 1 Stunde lang unter ständigem Rühren beziehungsweise Schütteln und unter Rückfluß zum Sieden erhitzt. Dann wurde das Lösungsmittel abgedampft, der Rückstand in Chloroform aufgenommen und die organische Phase mehrere Male mit Wasser gewaschen und darauffolgend über Magnesiumsulfat getrocknet. Nach dem Eindampfen des Lösungsmittels wurde der Rückstand aus Äthanol umkristallisiert. Ausbeute: 2,65 g (78% der Theorie) 1,5-Bis-[4'-(chlor)-phenyl]-3-[propylthio]-1,2,4-triazol mit einem Schmelzpunkt von 67 bis 68°C.

Weitere erfindungsgemäße 1,2,4-Triazolderivate der allgemeinen Formel I, welche nach der Verfahrensweise des vorstehenden Beispieles 1 hergestellt wurden, sind in der folgenden Tabelle 2 zusammengestellt.

Tabelle 2

| Beispiel Nr. | Verbindung | Ausbeute in % | Schmelzpunkt in °C |
|---|---|---|---|
| 2 | 1,5-Bis-[4'-(methoxy)-phenyl]-3-[methylthio]-1,2,4-triazol | 50,5 | 118 bis 120 |
| 3 | 1,5-Bis-[4'-(chlor)-phenyl]-3-[2"-(hydroxy)-äthylthio]-1,2,4-triazol | 85 | 100 bis 101 |
| 4 | 1,5-Bis-[4'-(chlor)-phenyl]-3-[pyrid-2"-ylmethylthio]-1,2,4-triazol | 83 | 134 bis 136 |
| 5 | 1,5-Bis-[4'-(chlor)-phenyl]-3-[pyrid-3"-ylmethylthio]-1,2,4-triazol | 72 | 155 bis 157 |
| 6 | 1-[4'-(Chlor)-phenyl]-5-[2'-(methylthio)-phenyl]-3-[pyrid-3"-ylmethylthio]-1,2,4-triazol | 65 | 111 bis 112 |
| 7 | 1,5-Bis-[4'-(chlor)-phenyl]-3-[4"-(fluor)-benzylthio]-1,2,4-triazol | 67 | 92 bis 93 |
| 8 | 1,5-Bis-[4'-(chlor)-phenyl]-3-[3",4"-di-(chlor)-benzylthio]-1,2,4-triazol | 74 | 128 bis 129 |
| 9 | 1,5-Bis-[4'-(chlor)-phenyl]-3-[äthoxycarbonylmethylthio]-1,2,4-triazol | 79 | 63 bis 64 |
| 10 | 1,5-Bis-[phenyl]-3-[cyanmethylthio]-1,2,4-triazol | 49 | 121 bis 122 |
| 11 | 1,5-Bis-[phenyl]-3-[äthoxycarbonylmethylthio]-1,2,4-triazol | 71 | 57 bis 58 |

Fortsetzung der Tabelle 2

| Beispiel Nr. | Verbindung | Ausbeute in % | Schmelzpunkt in °C |
|---|---|---|---|
| 12 | 1,5-Bis-[phenyl]-3-[1'-(äthoxycarbonyl)-äth-1'-ylthio)-1,2,4-triazol | 78 | 52 bis 53 |
| 13 | 1,5-Bis-[phenyl]-3-[2'-(oxo)-propylthio)-1,2,4-triazol | 76,5 | 83 bis 84 |
| 14 | 1-[4'-(Chlor)-phenyl]-5-[2"-(methylthio)-phenyl]-3-[methylthio]-1,2,4-triazol | 82 | 93 bis 94 |
| 15 | 1-[3'-(Trifluormethyl)-phenyl]-5-[2"-(methylthio)-phenyl]-3-[methylthio]-1,2,4-triazol | 85 | Öl |
| 16 | 1-[4'-(Fluor)-phenyl]-5-[2"-(methylthio)-phenyl]-3-[methylthio]-1,2,4-triazol | 68 | 73 bis 75 |
| 17 | 1-[Naphth-2'-yl]-5-[4"-(chlor)-phenyl]-3-[methylthio]-1,2,4-triazol | 55 | 124 bis 125 |
| 18 | 1-[4'-(Brom)-phenyl]-5-[4"-(chlor)-phenyl]-3-[methylthio]-1,2,4-triazol | 77 | 112 bis 113 |
| 19 | 1,5-Bis-[4'-(chlor)-phenyl]-3-[äthoxycarbonylthio]-1,2,4-triazol | 56 | Öl |

Fortsetzung der Tabelle 2

| Beispiel Nr. | Verbindung | Ausbeute in % | Schmelzpunkt in °C |
|---|---|---|---|
| 20 | 1,5-Bis-[4'-(chlor)-phenyl]-3-[methoxycarbonylthio]-1,2,4-triazol | 63,5 | Öl |
| 21 | 1,5-Bis-[4'-(chlor)-phenyl]-3-[äthylthio]-1,2,4-triazol | 66 | 124 bis 125 |
| 22 | 1,5-Bis-[4'-(chlor)-phenyl]-3-[2"-(oxo)-propylthio]-1,2,4-triazol | 57 | 96 bis 97 |
| 23 | 1,5-Bis-[4'-(chlor)-phenyl]-3-[pyrid-2"-yläthylthio]-1,2,4-triazol | 42 | 142 bis 143 |
| 24 | 1,5-Bis-[4'-(chlor)-phenyl]-3-[2",2",2"-tri-(fluor)-äth-1"-ylthio]-1,2,4-triazol | 45 | 80 bis 81 |
| 25 | 1-[4'-(Chlor)-phenyl]-5-[4"-(fluor)-phenyl]-3-(2''',2''',2'''-tri-(fluor)-äth-1'''-ylthio)-1,2,4-triazol | 63 | 54 bis 55 |
| 26 | 1,5-Bis-[4'-(fluor)-phenyl]-3-[2",2",2"-tri-(fluor)-äth-1"-ylthio]-1,2,4-triazol | 71 | 70 bis 71 |

Beispiel 27

1,5-Bis-[4'-(chlor)-phenyl]-3-
-[methylthio]-1,2,4-triazol

Verfahrensweise a)

Es wurden einer Lösung von 20,0 g (62,07 mMol) 1,5-Bis-[4'-(chlor)-phenyl]-1,2,4-triazolin-3(2H)-thion in 70 ml einer 5,0 g (125 mMol) Natriumhydroxyd enthaltenden wäßrigen Natriumhydroxydlösung und 150 ml Methanol unter ständigem Rühren beziehungsweise Schütteln während 10 Minuten 8 g (63,4 mMol) Dimethylsulfat zugetropft. Das Gemisch wurde 1 Stunde lang bei 60°C gerührt beziehungsweise geschüttelt und dann über Nacht stehengelassen. Das Lösungsmittel wurde abgedampft und der Rückstand wurde mit Chloroform extrahiert. Der Auszug wurde getrocknet, das Lösungsmittel wurde abgedampft und der Rückstand wurde aus Äthanol umkristallisiert. Ausbeute: 15,0 g (72% der Theorie) 1,5-Bis-[4'-(chlor)-phenyl]-3-[methylthio]-1,2,4-triazol mit einem Schmelzpunkt von 122 bis 123°C.

Verfahrensweise b)

Es wurden einer Suspension von 2 g (6,2 mMol) 1,5-Bis-[4'-(chlor)-phenyl]-1,2,4-triazolin-3(2H)-thion in 30 ml Äther während 10 Minuten unter ständigem Rühren beziehungsweise Schütteln bei Raumtemperatur 25 ml einer 12 gew.-%-igen ätherischen Lösung von Diazomethan zugetropft. Dieser Arbeitsgang wurde nach 8 Stunden wiederholt und dann wurde das Gemisch noch bei Raumtemperatur über Nacht weitergerührt beziehungsweise -geschüttelt. Danach wurden 20 ml einer 10 gew.-%-igen alkoholischen Chlorwasserstofflösung zugetropft, das Lösungsmittel wurde abgedampft und der Rückstand wurde aus Äthanol umkristallisiert. Ausbeute: 1,5 g (72% der Theorie) 1,5-Bis-[4'-(chlor)-phenyl]-3-

-[methylthio]-1,2,4-triazol mit einem Schmelzpunkt von 121 bis 123°C.

Weitere erfindungsgemäße 1,2,4-Triazolderivate der allgemeinen Formel I, welche nach den Verfahrensweisen des obigen Beispieles 27 hergestellt wurden, sind in der folgenden Tabelle 3 zusammengestellt.

Tabelle 3

| Beispiel Nr. | Verbindung | Ausbeute in % | Schmelzpunkt in °C |
|---|---|---|---|
| 28 | 1-[4'-(Chlor)-phenyl]-5-[2"-(fluor)-phenyl]-3-[methylthio]-1,2,4-triazol | 56 | 69 bis 71 |
| 29 | 1-[4'-(Chlor)-phenyl]-5-[2"-(chlor)-phenyl]-3-[methylthio]-1,2,4-triazol | 47 | 80 bis 83 |
| 30 | 1-[4'-(Chlor)-phenyl]-5-[thien-2"-yl]-3-[methylthio]-1,2,4-triazol | 80,5 | 129 bis 131 |
| 31 | 1-[4'-(Chlor)-phenyl]-5-[4"-(methoxy)-phenyl]-3-[methylthio]-1,2,4-triazol | 64 | 117 bis 119 |
| 32 | 1-[2'-(chlor)-phenyl]-5-[2"-(chlor)-phenyl]-3-[methylthio]-1,2,4-triazol | 86 | 118 bis 120 |
| 33 | 1-[2'-(Chlor)-phenyl]-5-[thien-2"-yl]-3-[methylthio]-1,2,4-triazol | 35 | 114 bis 117 |
| 34 | 1-[4'-(Fluor)-phenyl]-5-[4"-(methoxy)-phenyl]-3-[methylthio]-1,2,4-triazol | 42,5 | 103 bis 105 |
| 35 | 1-[4'-(Fluor)-phenyl]-5-[thien-2"-yl]-3-[methylthio]-1,2,4-triazol | 48 | 109 bis 111 |

Fortsetzung der Tabelle 3

| Beispiel Nr. | Verbindung | Ausbeute in % | Schmelzpunkt in °C |
|---|---|---|---|
| 36 | 1-[4'-(Fluor)-phenyl]-5-[2"-(methylsulfonyl)-phenyl]--3-[methylthio]-1,2,4-triazol | 47 | 144 bis 145 |
| 37 | 1-[4'-(Fluor)-phenyl]-5-[2"-(isopropylthio)-phenyl]--3-[methylthio]-1,2,4-triazol | 56 | 74 bis 75 |

## Beispiel 38

1-[4'-(Chlor)-phenyl]-5-[3"-(trifluormethyl)-phenyl]-3-[methylthio]-1,2,4-triazol

Es wurden einem Gemisch von 3,62 g (10 mMol) 1-[4'-(Chlor)-phenyl]-1-[3'-(trifluormethyl)-benzoyl]-thiosemicarbazid und einer Lösung von 1,2 g (30 mMol) Natriumhydroxyd in 24 ml Wasser und 48 ml Methanol unter ständigem Rühren beziehungsweise Schütteln 1,58 g (12,5 mMol) Dimethylsulfat zugetropft und es wurde 2 Stunden lang bei 60°C gerührt beziehungsweise geschüttelt. Nach dem Abdampfen des Methanoles wurde der Rückstand mit Chloroform extrahiert, der Auszug über Magnesiumsulfat getrocknet und eingedampft und der Rückstand aus Äthanol umkristallisiert. Ausbeute: 3,3 g (88% der Theorie) 1-[4'-(Chlor)-phenyl]-5-[3"-(trifluormethyl)-phenyl]-3-[methylthio]-1,2,4-triazol mit einem Schmelzpunkt von 108 bis 110°C.

Weitere erfindungsgemäße 1,2,4-Triazolderivate der allgemeinen Formel I, welche nach der Verfahrensweise des obigen Beispieles 38 hergestellt wurden, sind in der folgenden Tabelle 4 zusammengestellt.

Tabelle 4

| Beispiel Nr. | Verbindung | Ausbeute in % | Schmelzpunkt in °C |
|---|---|---|---|
| 39 | 1-[Phenyl]-5-[4'-(fluor)-phenyl]-3-[methylthio]-1,2,4-triazol | 76 | 89 bis 91 |
| 40 | 1-[4'-(Chlor)-phenyl]-5-[3",4"-di-(chlor)-phenyl]-3-[methylthio]-1,2,4-triazol | 75 | 119 bis 121 |
| 41 | 1-[4'-(Fluor)-phenyl]-5-[4"-(fluor)-phenyl]-3-[methylthio]-1,2,4-triazol | 70 | 81 bis 82 |
| 42 | 1-[3'-(Trifluormethyl)-phenyl]-5-[4"-(Chlor)-phenyl]-3-[methylthio]-1,2,4-triazol | 70 | 76 bis 78 |
| 43 | 1-[Phenyl]-5-[4'-(nitro)-phenyl]-3-[methylthio]-1,2,4-triazol | 77 | 142 bis 144 |
| 44 | 1-[4'-(Chlor)-phenyl]-5-[4"-(fluor)-phenyl]-3-[methylthio]-1,2,4-triazol | 78 | 84 bis 85 |
| 45 | 1-[4'-(Fluor)-phenyl]-5-[4"-(trifluormethyl)-phenyl]-3-[methylthio]-1,2,4-triazol | 81 | 123 bis 124 |
| 46 | 1-[4'-(Fluor)-phenyl]-5-[phenyl]-3-[methylthio]-1,2,4-triazol | 65 | 73 bis 75 |

Fortsetzung der Tabelle 4

| Beispiel Nr. | Verbindung | Ausbeute in % | Schmelzpunkt in °C |
|---|---|---|---|
| 47 | 1-[3'-(Trifluormethyl)-phenyl]-5-[4"-(fluor)-phenyl]-3-[methylthio]-1,2,4-triazol | 72 | 50 bis 52 |
| 48 | 1-[4'-(Methyl)-phenyl]-5-[4'-(chlor)-phenyl]-3-[methylthio]-1,2,4-triazol | 76 | 117 bis 118 |
| 49 | 1-[4'-(Chlor)-phenyl]-5-[3"-(fluor)-phenyl]-3-[methylthio]-1,2,4-triazol | 86 | 57 bis 59 |
| 50 | 1-[4'-(Chlor)-phenyl]-5-[4"-(nitro)-phenyl]-3-[methylthio]-1,2,4-triazol | 62 | 118 bis 120 |
| 51 | 1-[4'-(Chlor)-phenyl]-5-[4"-(methylthio)-phenyl]-3-[methylthio]-1,2,4-triazol | 72 | 125 bis 127 |
| 52 | 1-[4'-(Methylsulfonyl)-phenyl]-5-[4"-(chlor)-phenyl]-3-[methylthio]-1,2,4-triazol | 79,5 | 184 bis 185 |
| 53 | 1-[3'-(Chlor)-phenyl]-5-[4"-(fluor)-phenyl]-3-[methylthio]-1,2,4-triazol | 67,5 | 67 bis 69 |
| 54 | 1-[4'-(Brom)-phenyl]-5-[4"-(chlor)-phenyl]-3-[methylthio]-1,2,4-triazol | 62,5 | 115 bis 116 |

- 32 -

Fortsetzung der Tabelle 4

| Beispiel Nr. | Verbindung | Ausbeute in % | Schmelzpunkt in °C |
|---|---|---|---|
| 55 | 1-[4'-(Fluor)-phenyl]-5-[4"-(chlor)-phenyl]-3--[methylthio]-1,2,4-triazol | 78 | 95 bis 97 |

Beispiel 56

1,5-Bis-[phenyl]-3-[2'-(chlor)-
-1',1',2'-tri-(fluor)-äth-1'-
-ylthio]-1,2,4-triazol

Es wurde in ein Gemisch von 2,0 g (7,9 mMol) 1,5-Bis-[phenyl]-1,2,4-triazolin-3(2H)-thion, 50 ml wasserfreiem Dimethylformamid und 0,18 ml (0,4 mMol) Benzyltrimethylammoniumhydroxyd {Triton® B} unter ständigem Rühren beziehungsweise Schütteln bei Raumtemperatur bis zur Sättigung 1,1,2-Tri-(fluor)-2-(chlor)-äthylen eingeleitet, dann wurde das Rühren beziehungsweise Schütteln noch 1 Stunde bei 40 bis 50°C fortgesetzt. Darauffolgend wurde das Reaktionsgemisch in 200 ml Wasser eingegossen, abgekühlt, filtriert, mit Wasser gewaschen und aus Äthanol umkristallisiert. Ausbeute: 2,68 g (87% der Theorie) mit einem Schmelzpunkt von 64 bis 66°C.

Auch die in den folgenden Beispielen 57 bis 59 erscheinenden 1,2,4-Triazolderivate der allgemeinen Formel I wurden nach der Verfahrensweise des vorstehenden Beispieles 56 hergestellt.

Beispiel 57

1,5-Bis-[phenyl]-3-[1',1',2',2'-
-tetra-(fluor)-äthylthio)-1,2,4-
-triazol

Ausbeute: 61% der Theorie,
Schmelzpunkt: 48 bis 49°C.

Beispiel 58

1,5-Bis-[4'-(chlor)-phenyl]-3-
-[1",1",2",2"-tetra-(fluor)-
-äthylthio]-1,2,4-triazol

Ausbeute: 59% der Theorie,
Schmelzpunkt: 91 bis 92°C.

Beispiel 59

1,5-Bis-[4'-(fluor)-phenyl]-3-
-[1",1",2",2"-tetra-(fluor)-
-äthylthio]-1,2,4-triazol

Ausbeute: 53% der Theorie,
Schmelzpunkt: 83 bis 86°C.

Beispiel 60

1,5-Bis-[4'-(fluor)-phenyl]-3-
-[2",2",3",3"-tetra-(fluor)-prop-
-1"-ylthio]-1,2,4-triazol

Es wurde eine Lösung von 3,07 g (10 mMol) 1-[4'-(Fluor)-phenyl]-1-[4"-(fluor)-benzoyl]-thiosemicarbazid und 0,64 g (16 mMol) Natriumhydroxyd in 6,4 ml Wasser zunächst mit 12,8 ml Methanol und dann mit 3,43 g (12 mMol) 1-[p-Toluolsulfonyloxy]-2,2,3,3-tetra-[fluor]-propan versetzt und das Reaktionsgemisch wurde 90 Minuten lang bei 50 bis 70°C gerührt beziehungsweise geschüttelt. Nach dem Abdampfen des Lösungsmittels wurde der Rückstand in Dichlormethan aufgenommen und der Auszug mit Wasser gewaschen, über Natriumsulfat getrocknet und eingedampft. Als Rückstand wurde 1,0 g (24% der Theorie) 1,5-Bis-[4'-(fluor)-phenyl]-3-[2",2",3",3"-tetra-(fluor)-prop-1"-ylthio]-1,2,4-triazol in Form eines nicht kristallisierbaren Öles erhalten.

## Beispiel 61

1,5-Bis-[phenyl]-3-[methylsulfinyl]-
-1,2,4-triazol

Es wurde einer Lösung von 0,58 g (2,17 mMol) 1,5-Bis-[phenyl]-3-[methylthio]-1,2,4-triazol in 10 ml wasserfreiem Chloroform eine Lösung von 0,42 g (2,2 mMol) m-(Chlor)-perbenzoesäure in 5 ml Chloroform zugetropft und das Gemisch wurde über Nacht bei Raumtemperatur gerührt beziehungsweise geschüttelt. Das Reaktionsgemisch wurde dann zunächst mit einer 6 gew.-%-igen Natriumbicarbonatlösung und dann mit Wasser gewaschen und über Natriumsulfat getrocknet. Ausbeute: 0,4 g (64% der Theorie) 1,5-Bis-[phenyl]-3-[methylsulfinyl]-1,2,4-triazol mit einem Schmelzpunkt von 121 bis 123°C (nach der Umkristallisation aus Äthanol).

Auch die in den folgenden Beispielen 62 und 63 erscheinenden 1,2,4-Triazolderivate der allgemeinen Formel I wurden nach der Verfahrensweise des vorstehenden Beispieles 61 hergestellt.

## Beispiel 62

1-[4'-(Chlor)-phenyl]-5-[4"-
-(fluor)-phenyl]-3-[methylsulfinyl]-
-1,2,4-triazol

Ausbeute: 39,5% der Theorie, Öl.

## Beispiel 63

1,5-Bis-[4'-(chlor)-phenyl]-3-
-[methylsulfinyl]-1,2,4-triazol

Ausbeute: 35% der Theorie,
Schmelzpunkt: 95 bis 96°C.

## Beispiel 64

1,5-Bis-[4'-(chlor)-phenyl]-3-
-[methylsulfonyl]-1,2,4-triazol

Es wurde einem Gemisch von 1,68 g (5 mMol) 1,5-Bis-[4'-(chlor)-phenyl]-3-[methylthio]-1,2,4-triazol und 17 ml wasserfreiem Chloroform, bei 0°C unter ständigem Rühren beziehungsweise Schütteln während 30 Minuten eine Lösung von 2,3 g (12 mMol) einer 90 gew.-%-igen m-(Chlor)-perbenzoesäure in 25 ml Chloroform zugetropft. Nach 2 Stunden wurde das Gemisch mit einer 6 gew.-%-igen wäßrigen Natriumbicarbonatlösung und dann mit Wasser gewaschen und über Magnesiumsulfat getrocknet. Ausbeute: 1,8 g (98% der Theorie) 1,5-Bis-[4'-(chlor)-phenyl]-3-[methylsulfonyl]-1,2,4-triazol mit einem Schmelzpunkt von 183 bis 185°C (nach der Umkristallisation aus Äthanol).

Weitere erfindungsgemäße 1,2,4-Triazolderivate der allgemeinen Formel I, welche nach der Verfahrensweise des Beispieles 64 hergestellt wurden, sind in der folgenden Tabelle 5 zusammengestellt.

Tabelle 5

| Beispiel Nr. | Verbindung | Ausbeute in % | Schmelzpunkt in °C |
|---|---|---|---|
| 65 | 1-[4'-(Chlor)-phenyl]-5-[4"-(fluor)-phenyl]-3--[methylsulfonyl]-1,2,4-triazol | 87 | 149 bis 150 |
| 66 | 1-[4'-(Fluor)-phenyl]-5-[4"-(chlor)-phenyl]-3--[methylsulfonyl]-1,2,4-triazol | 84 | 150 bis 151 |
| 67 | 1,5-Bis-[4'-(fluor)-phenyl]-3-[methylsulfonyl]--1,2,4-triazol | 73,5 | 160 bis 161 |
| 68 | 1,5-Bis-[phenyl]-3-[methylsulfonyl]-1,2,4-triazol | 50 | 131 bis 133 |
| 69 | 1,5-Bis-[4'-(fluor)-phenyl]-3-[2",2",2"-tri-(fluor)--äth-1"-ylsulfonyl]-1,2,4-triazol | 56 | 109 bis 110 |
| 70 | 1-[4'-(Chlor)-phenyl]-5-[4"-(fluor)-phenyl]-3--[pyrid-2'''-ylmethylsulfonyl]-1,2,4-triazol | 57 | 197 bis 199 |
| 71 | 1,5-Bis-[phenyl]-3-[1'1',2',2'-tetra-(fluor)--äthylsulfonyl]-1,2,4-triazol | 24 | 119 bis 122 |
| 72 | 1,5-Bis-[phenyl]-3-[1',1',2'-tri-(fluor)-2'-(chlor)--äth-1"-ylsulfonyl]-1,2,4-triazol | 47,5 | 118 bis 119 |

## Beispiel 73

### Herstellung von Tabletten

Zusammensetzung einer Tablette

| | |
|---|---|
| 1-[4'-(Chlor)-phenyl]-5-[4"-(fluor)-phenyl]-3-[methylthio]-1,2,4-triazol {Verbindung des Beispieles 44} | 150 mg |
| Mikrokristalline Cellulose [Elcema® P 100, Produkt der Firma Degussa, Bundesrepublik Deutschland] | 50 mg |
| Mikrokristalline Cellulose [Elcema® G 250, Produkt der Firma Degussa, Bundesrepublik Deutschland] | 40 mg |
| Talk | 7 mg |
| Magnesiumstearat | 3 mg |
| | 250 mg |

Patentansprüche

—

0155486

## Patentansprüche

1.) 1,3,5-Trisubstituierte 1,2,4-Triazolderivate der allgemeinen Formel

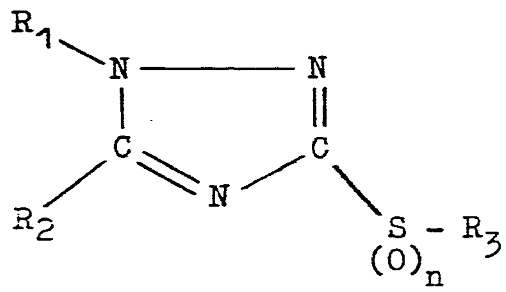

I ,

worin

$R_1$ für einen, gegebenenfalls durch 1 oder mehr Fluor-, Chlor- und/oder Bromatom(e), Alkylrest(e) mit 1 bis 4 Kohlenstoffatom(en), Alkoxyrest(e) mit 1 bis 4 Kohlenstoffatom(en), Nitrogruppe(n), Trifluormethylrest(e), Alkylthiorest(e) mit 1 bis 4 Kohlenstoffatom(en), Alkylsulfinylrest(e) mit 1 bis 4 Kohlenstoffatom(en) und/oder Alkylsulfonylrest(e) mit 1 bis 4 Kohlenstoffatom(en) substituierten, Phenylrest oder einen Naphthylrest steht,

$R_2$ einen, gegebenenfalls durch 1 oder mehr Fluor-, Chlor- und/oder Bromatom(e), Alkylrest(e) mit 1 bis 4 Kohlenstoffatom(en), Alkoxyrest(e) mit 1 bis 4 Kohlenstoffatom(en), Nitrogruppe(n), Trifluormethylrest(e), Alkylthiorest(e) mit 1 bis 4 Kohlenstoffatom(en), Alkylsulfinylrest(e) mit 1 bis 4 Kohlenstoffatom(en) und/oder Alkyl-

0155486

sulfonylrest(e) mit 1 bis 4 Kohlenstoffatom(en) substituierten, Phenylrest oder einen Thienylrest bedeutet,

$R_3$ einen, gegebenenfalls durch 1 oder mehr Fluor- und/oder Chloratom(e), Hydroxygruppe(n), Alkoxycarbonylrest(e) mit 2 bis 4 Kohlenstoffatomen, Cyangruppe(n) und/oder Pyridylrest(e) substituierten, Alkylrest mit 1 bis 4 Kohlenstoffatom(en), einen Alkoxycarbonylrest mit 2 bis 4 Kohlenstoffatomen, einen Oxoalkylrest mit 2 bis 5 Kohlenstoffatomen oder einen im Phenylring durch 1 oder mehr Fluor- und/oder Chloratom(e) substituierten Phenylalkylrest mit 1 bis 4 Kohlenstoffatom(en) im Alkylteil darstellen, und

n 0, 1 oder 2 ist,

mit der weiteren Maßgabe, daß,

im Falle daß

n 0 ist und

$R_3$ einen Methylrest bedeutet,

$R_1$ und $R_2$ nicht beide einen nicht substituierten Phenylrest darstellen.

2.) 1,2,4-Triazolderivate nach Anspruch 1, dadurch gekennzeichnet, daß der beziehungsweise die Alkylrest(e), Alkoxyrest(e), Alkylthiorest(e), Alkylsulfinylrest(e) und/oder Alkylsulfonylrest(e), durch welche[n] der beziehungsweise die Phenylrest(e), für den beziehungsweise die $R_1$ und/oder $R_2$ stehen kann beziehungsweise können, substituierte sein kann beziehungsweise können, [ein] solche[r] mit 1 bis 3, insbesondere 1 oder 3 Kohlenstoffatom(en) ist beziehungsweise sind.

3.) 1,2,4-Triazolderivate nach Anspruch 1 oder 2, dadurch gekennzeichnet, daß der Alkylrest, für welchen $R_3$ stehen kann, ein solcher mit 1 bis 3, insbesondere 1 oder 2, Kohlenstoffatom(en) ist.

4.) 1,2,4-Triazolderivate nach Anspruch 1 bis 3, dadurch gekennzeichnet, daß der Alkoxycarbonylrest, für den $R_3$ stehen kann, beziehungsweise der beziehungsweise die Alkoxycarbonylrest(e), durch welche[n] der Alkylrest, für den $R_3$ stehen kann, substituiert sein kann, [ein] solche[r] mit 2 oder 3, insbesondere 2, Kohlenstoffatomen ist beziehungsweise sind.

5.) 1,2,4-Triazolderivate nach Anspruch 1 bis 4, dadurch gekennzeichnet, daß der Oxoalkylrest, für den $R_3$ stehen kann, ein solcher mit 2 bis 4, insbesondere 2 oder 3, Kohlenstoffatom(en) ist.

6.) 1,2,4-Triazolderivate nach Anspruch 1 bis 4, dadurch gekennzeichnet, daß der Phenylalkylrest, für welchen $R_3$ stehen kann, ein solcher mit 1 bis 3, insbesondere 1 oder 2, Kohlenstoffatom(en) im Alkylteil ist.

7.) 1,5-Bis-[4'-(chlor)-phenyl]-3-[methylthio]-1,2,4-triazol, 1-[4'-(Chlor)-phenyl]-5-[4''-(fluor)-phenyl]-3-[methylthio]-1,2,4-triazol, 1-[4'-(Fluor)-phenyl]-5-[4''-(chlor)-phenyl]-3-[methylthio]-1,2,4-triazol, 1-[4'-(Chlor)-phenyl]-5-[4''-(fluor)-phenyl]-3-[methylsulfonyl]-1,2,4-triazol und 1,5-Bis-[4'-(fluor)-phenyl]-3-[methylsulfonyl]-1,2,4-triazol.

8.) Verfahren zur Herstellung der Verbindungen nach Anspruch 1 bis 7, dadurch gekennzeichnet, daß man

a) 1,5-disubstituierte 1,2,4-Triazolin-3[2H]-thione der allgemeinen Formel

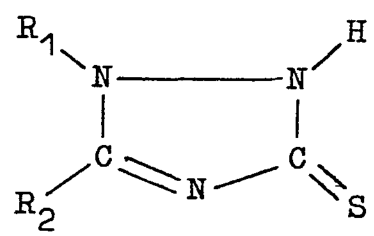

II ,

worin $R_1$ und $R_2$ die im Anspruch 1 oder 2 angegebenen Bedeutungen haben, oder 1-substituierte 1-(Acyl)-thiosemicarbazide der allgemeinen Formel

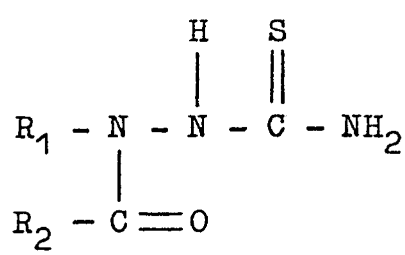

V ,

worin $R_1$ und $R_2$ die im Anspruch 1 oder 2 angegebenen Bedeutungen haben, in Lösung in inerten Lösungsmitteln in Gegenwart von organischen und/oder anorganischen Basen im alkalischen pH-Bereich mit Verbindungen der allgemeinen Formel

$R_3 - X$ III ,

worin $R_3$ die in den Ansprüchen 1 oder 3 bis 6 angegebenen Bedeutungen hat und X ein Halogenatom oder einen Toluolsulfonyloxyrest bedeutet, umsetzt und gegebenenfalls die erhaltenen 1,2,4-Triazolderivate der allgemeinen Formel I, bei welchen n 0 ist, zu 1,2,4-Triazolderivaten der allgemeinen Formel I, bei welchen n 1 oder 2 ist, oxydiert oder

b) zur Herstellung derjenigen 1,2,4-Triazolderivate der allgemeinen Formel I, bei welchen $R_3$ für einen durch 1 oder mehr Fluor- und/oder Chloratom(e) substituierten Äthylrest steht und n 0 ist, Tetrahalogenäthylene der allgemeinen Formel

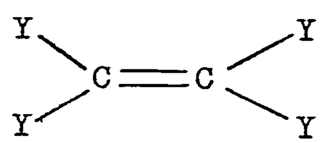

IV ,

worin Y unabhängig voneinander Fluor- und/oder Chloratome bedeuten, an 1,5-disubstituierte 1,2,4-Triazolin-3[2H]-thione der allgemeinen Formel II oder 1-substituierte 1-(Acyl)-thiosemicarbazide der allgemeinen Formel V in inerten Lösungsmitteln in Gegenwart von organischen und/oder anorganischen Basen im alkalischen pH-Bereich anlagert oder

c) zur Herstellung derjenigen 1,2,4-Triazolderivate der allgemeinen Formel I, bei welchen $R_3$ für einen Alkylrest steht und n 0 ist, 1,5-disubstituierte 1,2,4-Triazolin-3[2H]-thione der

allgemeinen Formel II oder 1-substituierte 1-(Acyl)-thiosemicarbazide der allgemeinen Formel V in inerten Lösungsmitteln, in Gegenwart von organischen und/oder anorganischen Basen im alkalischen pH-Bereich mit Diazomethan oder Dialkylsulfaten mit 1 bis 4 Kohlenstoffatom(en) alkyliert.

9.) Arzneimittel, gekennzeichnet durch einen Gehalt an 1 oder mehr Verbindung(en) nach Anspruch 1 bis 7 als Wirkstoff(en), zweckmäßigerweise zusammen mit 1 oder mehr üblichen inerten nicht toxischen festen oder flüssigen Trägerstoff(en), Hilfsmittel(n) und/oder Zusatzstoff(en).

Zusammenfassung

0155486

Nummer der Anmeldung

Europäisches Patentamt

# EUROPÄISCHER RECHERCHENBERICHT

EP 85 10 1280

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | CHEMICAL ABSTRACTS, Band 25, nr. 9, 10. Mai 1931, Seiten 2119-2120, Columbus, Ohio, US; SATISH CHANDRA DE u.a.: "Oxidation. IV. Action of ferric chloride and hydrogen peroxide on S-alkylthio-semicarbazones. Formation of triazoles" & J. INDIAN CHEM. SOC. 7, 875-8(1930); & TRIAZOLES 1,2,4, von Carroll Temple, Jr., Seiten 278,281 und 282, John Wiley & Sons, New York.<br>--- | 1-3 | C 07 D 249/12<br>C 07 D 409/04<br>C 07 D 401/12<br>A 61 K 31/41<br>A 61 K 31/44 //<br>(C 07 D 409/04<br>C 07 D 333:00<br>C 07 D 249:00 )<br>(C 07 D 401/12<br>C 07 D 249:00<br>C 07 D 213:00 ) |
| A | FR-M- 1 403 (LABORATORIOS MIQUEL)<br>----- | | |
| | | | RECHERCHIERTE SACHGEBIETE (Int. Cl.4) |
| | | | C 07 D 249/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 09-05-1985 | THEUNS H.G. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82